Europäisches Patentamt

(19) European Patent Office

Office européen des brevets

(11) Veröffentlichungsnummer: **0 078 955**
A1

(12) # EUROPÄISCHE PATENTANMELDUNG

(21) Anmeldenummer: 82109769.8

(22) Anmeldetag: 22.10.82

(51) Int. Cl.³: **C 07 C 121/34**
C 07 C 69/67

(30) Priorität: 26.10.81 DE 3142423

(43) Veröffentlichungstag der Anmeldung:
18.05.83 Patentblatt 83/20

(84) Benannte Vertragsstaaten:
BE CH DE FR GB IT LI NL

(71) Anmelder: HOECHST AKTIENGESELLSCHAFT
Postfach 80 03 20
D-6230 Frankfurt/Main 80(DE)

(72) Erfinder: Mack, Karl Ernst, Dr.
Klingenbachstrasse 43
D-6200 Wiesbaden(DE)

(72) Erfinder: Müller, Werner Heinrich, Dr.
Taunusblick 5
D-6239 Eppstein/Taunus(DE)

(54) Verfahren zur Herstellung von Estern oder Nitrilen von 5-Oxoalkansäuren.

(57) Die Erfindung betrifft ein Verfahren zur Herstellung der Ester oder Nitrile von 5-Oxoalkansäuren durch Umsetzung eines Ketons, das mindestens ein aktives H-Atom in α-Position besitzt, mit einem Ester oder dem Nitril einer α,β-ungesättigten Carbonsäure. Als Katalysator wird Ammoniak und/oder ein Ammoniumsalz eingesetzt.

EP 0 078 955 A1

Croydon Printing Company Ltd.

HOECHST AKTIENGESELLSCHAFT     Dr.MA/AK     HOE 81/F 286

Verfahren zur Herstellung von Estern oder Nitrilen von
5-Oxoalkansäuren

Das erfindungsgemäße Verfahren betrifft die Herstellung von Estern oder Nitrilen von 5-Oxoalkansäuren durch Umsetzung eines Ketons, das mindestens ein aktives H-Atom in $\alpha$-Position besitzt, mit einem Ester oder dem Nitril einer $\alpha,\beta$-ungesättigten Carbonsäure. Die bei dieser als Michaeladdition bezeichneten Reaktion entstehenden Ester oder Nitrile der 5-Oxoalkansäuren sind Ausgangsmaterialien zur Herstellung wichtiger Produkte. So kann z.B. das Additionsprodukt aus Cyclohexanon und Acrylsäureester durch anschließende Cyclisierung zu Dihydrocumarin, einem wertvollen Ausgangsmaterial für Aromastoffe, umgewandelt werden. Das Additionsprodukt aus Aceton und Acrylnitril kann in $\alpha$-Picolin, das Additionsprodukt aus Aceton und Acrylsäureester zu Resorcin, einem begehrten Produkt zur Herstellung z.B von Kunstharzen, umgewandelt werden.

Es ist bekannt, die Ester oder Nitrile von 5-Oxoalkansäuren aus den obengenannten Ausgangsstoffen herzustellen indem primäre Amine oder Schiff'sche Basen (DE-PS 2 329 923, DE-OS 2 325 160, DE-OS 2 355 859, DE-PS 2 540 072), oder sekundäre Amine (EP 0 008 428 A1) als Katalysatoren eingesetzt werden.

Es hat sich nun überraschenderweise herausgestellt, daß man die häufig schwer zugänglichen und daher teuren Amine und Schiff'schen Basen durch - den großtechnisch erzeugten, leicht zugänglichen und daher ökonomisch günstigeren Ammoniak bzw. durch Ammoniumsalze ersetzen kann, die außerdem weniger giftig sind als die meisten der genannten Stickstoffverbindungen.

Das erfindungsgemäße Verfahren zur Herstellung der Ester oder Nitrile von 5-Oxoalkansäuren durch Umsetzung eines Ketons, das mindestens ein aktives H-Atom in $\alpha$-Position besitzt, mit einem Ester oder dem Nitril einer $\alpha$,ß-ungesättigten Carbonsäure ist dadurch gekennzeichnet, daß die Umsetzung in Gegenwart von Ammoniak und/oder einem Ammoniumsalz durchgeführt wird.

Geeignete Ketone sind z.B. Aceton, Methylethylketon, Diethylketon, Methylisopropylketon, Acetophenon, Cyclopentanon und Cyclohexanon. Bevorzugt werden nach dem erfindungsgemäßen Verfahren Aceton, Methylethylketon oder Diethylketon umgesetzt. Besonders bevorzugt ist Aceton.

Von den Estern und Nitrilen der $\alpha$,ß-ungesättigten Carbonsäuren sind z.B. die von Acrylsäure, Methacrylsäure und Crotonsäure abgeleiteten von praktischer Bedeutung. Bevorzugt werden der Methyl- oder Ethylester oder die Nitrile dieser drei Säuren eingesetzt. Besonders bevorzugt ist der Methylester und das Nitril der Acrylsäure.

Das molare Verhältnis von Keton zu Ester bzw. Nitril beträgt im allgemeinen 1 : 1 bis 20 : 1, bevorzugt 3 : 1 bis 8 : 1. Ein Zusatz von Lösemittel ist im allgemeinen nicht erforderlich, da vorzugsweise das Keton im Überschuß verwendet wird. Um die Polymerisation der ungesättigten Verbindung während der Reaktion zu verhindern, ist es günstig, einen Polymerisationsinhibitor wie Hydrochinon zuzusetzen.

Als Katalysator wird Ammoniak und/oder ein Ammoniumsalz verwendet. Geeignete Ammoniumsalze sind z.B. solche, die sich von Halogenwasserstoffsäuren, Phosphorsäure oder Carbonsäuren ableiten, und schließlich solche, die sich von derselben $\alpha$ ,ß-ungesättigten Carbonsäure ableiten wie die eingesetzten Ester oder Nirile (z.B. Ammoniumacrylat oder Ammoniumcrotonat) bzw. solche, die sich von derselben 5-Oxoalkansäure ableiten wie die bei der erfindungsgemäßen Reaktion hergestellten Ester bzw. Nitrile (wie das Ammoniumsalz der 5-Oxohexansäure). Das Ammoniumsalz kann entweder als solches dem Einsatzgemisch hinzugefügt werden oder aber durch Zusatz von Ammoniak und der entsprechenden Säure zum Einsatzgemisch in situ erzeugt werden. Bei der Verwendung von Ammoniak mit einem Zusatz einer geringen Menge eines Ammoniumsalzes ist es günstig, das Ammoniumsalz in situ zu erzeugen, indem zum Ammoniak enthaltenden Einsatzgemisch eine geringe Menge Säure zugesetzt wird. Bei der Verwendung von Ammoniak mit einer größeren Menge Ammoniumsalz oder bei der alleinigen Verwendung von Ammoniumsalz hingegen ist es günstig, das Ammoniumsalz als solches dem Einsatzgemisch hinzuzufügen. Von den genannten Katalysatoren erweist sich die Verwendung von Ammoniak unter Zusatz geringer Mengen von Ammoniumacetat, Ammoniumacrylat, Ammoniumfluorid oder Ammoniumbenzoat oder dem Ammoniumsalz der 5-Oxoalkansäure als besonders günstig, vor allem aber Ammoniak unter Zusatz von geringen Mengen an Ammoniumbenzoat oder dem Ammoniumsalz der 5-Oxohexansäure.

Die Gesamtmenge an Ammoniak und/oder Ammoniumsalz beträgt 1 bis 50 Mol%, bevorzugt 5 bis 15 Mol%, bezogen auf eingesetzten Ester oder Nitril. Bei der bevorzugten Verwendung von Ammoniak unter Zusatz einer geringen Menge Ammoniumsalz ist die Gesamtmenge wie bereits angegeben und die Ammoniumsalzmenge 0,1 bis 1 Mol%, bezogen auf den eingesetzten Ester oder das Nitril.

Es erweist sich aus praktischen Gründen, z.B. im Hinblick auf die Dosierung des Katalysators, als günstig, Ammoniak und/oder die Ammoniumsalze als wäßrige Lösung - vorzugsweise eine bei Raumtemperatur gesättigte Lösung - zu verwenden. Die dadurch bedingte Anwesenheit von etwa 1 - 10 Gew.% Wasser im Einsatzgemisch wirkt sich außerdem im allgemeinen günstig auf die Umsetzung aus.

Das erfindungsgemäße Verfahren wird im allgemeinen bei Temperaturen von 100 bis 250°C, bevorzugt bei 180 bis 230°C durchgeführt. Der Druck hat geringen Einfluß auf die Reaktion, soll aber mindestens so hoch eingestellt werden - ggf. unter Zusatz von Inertgas - daß die Reaktion in flüssiger Phase stattfindet.

Die folgenden Beispiele sollen die Erfindung näher erläutern.

Beispiele 1 und 2

Jeweils 160 ml eines Gemisches aus Aceton (AC) und Acrylnitril (AN) im molaren Verhältnis 3 : 1 werden mit 0,1 g Hydrochinon und 10 Mol% Ammoniak,bezogen auf Acrylnitril, versetzt. Der Ammoniak wird in Beispiel 1 gasförmig eingeleitet und in Beispiel 2 als 25 %ige wäßrige Lösung zugegeben. Diese Einsatzgemische werden in einem Autoklaven (Volumen 200 ml) während 0,5 Stunden auf 225°C erhitzt. Die Reaktionsprodukte werden gaschromatographisch analysiert.

Tabelle 1 zeigt die erreichten Umsätze an Acrylnitril und die Selektivität der Bildung von 5-Oxohexansäurenitril (OHN) bezogen auf umgesetztes Acrylnitril.

Tabelle 1    Umsetzung von Aceton und Acrylnitril mit Ammoniak

| Beispiel Nr. | Ammoniak | % Umsatz AN | % Selektivität OHN |
|---|---|---|---|
| 1 | gasförmig | 70 | 53 |
| 2 | wäßrig | 65 | 62 |

Beispiele 3 und 4

Jeweils 160 ml eines Gemischs aus Aceton und Acrylnitril im molaren Verhältnis 3 : 1 werden mit 0,1 g Hydrochinon und einer wäßrigen Lösung an Ammoniumfluorid (Beispiel 3) bzw. von Ammoniumacetat (Beispiel 4) in einem Autoklaven (Volumen 200 ml) während 2 Stunden auf 230°C erhitzt. Das Reaktionsprodukt wird gaschromatographisch analysiert.

Tabelle 2 enthält die jeweils verwendete molare Menge des Ammoniumsalzes (bezogen auf Acrylnitril), die Wassermenge in Gew. %, bezogen auf Aceton und Acrylnitril, sowie die erzielten Umsätze an Acrylnitril (AN) und die Selektivität der Bildung von 5-Oxohexansäurenitril (OHN), bezogen auf umgesetztes Acrylnitril.

Tabelle 2  Umsetzung von Aceton und Acrylnitril mit
Ammoniumsalzen .

| Beispiel Nr. | Ammonium- salz | Mol% Ammoniumsalz | Gew.% Wasser | %-Umsatz AN | %-Selektivität OHN |
|---|---|---|---|---|---|
| 3 | Ammonium- fluorid | 5 | 10 | 72 | 66 |
| 4 | Ammonium- acetat | 10 | 5 | 78 | 68 |

## Beispiele 5 - 8

Jeweils 160 ml eines Gemischs aus Aceton (AC) und Acrylnitril (AN) in unterschiedlichen molaren Verhältnissen
(s. Tabelle), denen weiterhin 0,1 g Hydrochinon und 0,3 g
Benzoesäure sowie 25 %iger, wäßriger Ammoniak in unterschiedlichen Mengen (s. Tabelle) zugefügt wurden, werden
in einem Autoklaven (Volumen 200 ml) während unterschiedlicher Dauer (s. Tabelle) auf 225°C erhitzt. Die Reaktionsprodukte werden gaschromatographisch analysiert.

Tabelle 3 zeigt die erzielten Selektivitäten der Bildung
von 5-Oxohexansäurenitril (OHN), bezogen auf umgesetztes
Acrylnitril, sowie den Umsatz an Acrylnitril.

## Tabelle 3·

| Beispiel Nr. | molares Ver- hältnis AC : AN | Mol% $NH_3$ bezogen auf AN | Reaktionszeit (Stunden) | %-Umsatz AN | %-Selektivität OHN |
|---|---|---|---|---|---|
| 5 | 3 : 1 | 10 | 1/2 | 58 | 76 |
| 6 | 5 : 1 | 10 | 1/2 | 57 | 84 |
| 7 | 3 : 1 | 5 | 1/2 | 43 | 83 |
| 8 | 3 : 1 | 10 | 1 | 82 | 74 |

Beispiel 9

160 ml eines Gemisches aus Aceton und Acrylsäuremethylester im molaren Verhältnis 5 : 1 werden mit 0,1 g Hydrochinon, 0,3 g Benzoesäure und 10 Mol% (bezogen auf Acrylsäuremethylester) 25 %igem wäßrigem Ammoniak versetzt und in einem Autoklaven (Volumen 200 ml) während 0,5 Stunden auf 230°C erhitzt. Aus der gaschromatographischen Analyse des Reaktionsprodukts ergibt sich ein Umsatz an Acrylsäuremethylester von 65 %. Die Bildung von 5-Oxohexansäuremethylester erfolgt mit einer Selektivität von 83 %, bezogen auf den umgesetzten Acrylsäuremethylester.

0078955

Patentansprüche                          HOE 81/F 286

1. Verfahren zur Herstellung der Ester oder Nitrile von 5-Oxoalkansäuren durch Umsetzung eines Ketons, das mindestens ein aktives H-Atom in $\alpha$-Position besitzt, mit einem Ester oder dem Nitril einer $\alpha,\beta$-ungesättigten Carbonsäure, dadurch gekennzeichnet, daß die Umsetzung in Gegenwart von Ammoniak und/oder einem Ammoniumsalz durchgeführt wird.

2. Verfahren nach Anspruch 1, dadurch gekennzeichnet, daß Ammoniak und eine geringe Menge Ammoniumsalz verwendet werden.

3. Verfahren nach Anspruch 1 oder 2, dadurch gekennzeichnet, daß als Keton Aceton, Methylethylketon, Diethylketon, Methylisopropylketon, Acetophenon, Cyclopentanon oder Cyclohexanon verwendet werden.

4. Verfahren nach einem der Ansprüche 1 bis 3, dadurch gekennzeichnet, daß das Nitril, der Methyl- oder Ethylester der Acrylsäure, Methacrylsäure oder Crotonsäure verwendet werden.

5. Verfahren nach einem der Ansprüche 1 bis 4, dadurch gekennzeichnet, daß Aceton mit Acrylsäuremethylester oder mit Acrylnitril umgesetzt wird.

6. Verfahren nach einem der Ansprüche 1 bis 5, dadurch gekennzeichnet, daß die Reaktionstemperatur 100 bis 250°C beträgt.

7. Verfahren nach einem der Ansprüche 1 bis 6, dadurch gekennzeichnet, daß Aceton mit Acrylnitril oder Acrylsäuremethylester in Gegenwart von wäßrigem Ammoniak und einer geringen Menge Ammoniumbenzoat bei 200 bis 230°C umgesetzt wird.

# EUROPÄISCHER RECHERCHENBERICHT

**0078955**
Nummer der Anmeldung

EP 82 10 9769

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int. Cl. ³) |
|---|---|---|---|
| | --- | | C 07 C 121/34 |
| A | EP-A-0 013 254 (HOECHST) | | C 07 C 69/67 |
| | * Vollständiges Dokukment * | | |
| | --- | | |
| A | EP-A-0 008 482 (STAMICARBON) | | |
| | * Vollständiges Dokument * | | |
| | ----- | | |

**RECHERCHIERTE SACHGEBIETE (Int. Cl ³)**

C 07 C 69/00
C 07 C 121/34

Der vorliegende Recherchenbericht wurde für alle Patentansprüche erstellt.

| Recherchenort | Abschlußdatum der Recherche | Prüfer |
|---|---|---|
| BERLIN | 19-01-1983 | BREW C.H. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82